# EUROPEAN PATENT SPECIFICATION

(11) **EP 4 554 662 B1**
(45) Date of publication and mention of the grant of the patent: **30.09.2026**
(21) Application number: 23840579.9
(22) Date of filing: 14.07.2023
(51) Int. Cl.: A61N 1/36, A61N 1/378, A61N 1/05

(54) **SYSTEMS FOR DELIVERING NEUROSTIMULATION, INCLUDING REDUCED DOSES OF NEUROSTIMULATION**
SYSTEME ZUR ABGABE VON NEUROSTIMULATION MIT VERRINGERTEN DOSEN VON NEUROSTIMULATION
SYSTÈMES D'ADMINISTRATION DE NEUROSTIMULATION, COMPRENANT DES DOSES RÉDUITES DE NEUROSTIMULATION

(30) Priority: 15.07.2022 US 202263389747 P
(43) Date of publication of application: 21.05.2025
(73) Proprietor: Nevro Corporation, Redwood City, CA 94065 (US)
(72) Inventor: PANNU, Satinderpall, Singh, Pleasanton, CA 94588 (US)
(74) Representative: Morabito, Sara
(86) International application number: PCT/US2023/070250
(87) International publication number: WO 2024/015987

(56) References cited:
- WO-A2-2021/016616
- CN-A- 114 555 177
- JP-A- 2020 518 398
- US-A1- 2006 069 415
- US-A1- 2017 087 369
- US-A1- 2017 361 089
- US-A1- 2019 046 800
- US-A1- 2021 031 033
- US-A1- 2022 143 403
- US-A1- 2022 143 403
- US-B1- 10 300 277

## Description

### CROSS-REFERENCE TO RELATED APPLICATION(S)

The present application claims priority to U.S. Provisional Patent Application No. 63/389,747, filed July 15, 2022.

### TECHNICAL FIELD

The present technology is directed toward electrically modulating nervous tissue to treat a patient condition.

### BACKGROUND

Neurological stimulators have been developed to treat pain, movement disorders, functional disorders, spasticity, cancer, cardiac disorders, and various other medical conditions. Neurological stimulation systems generally have a signal generator and one or more implantable leads that deliver electrical pulses to neurological tissue or muscle tissue. For example, several neurological stimulation systems for spinal cord stimulation (SCS) have cylindrical leads that include a lead body with a circular cross-sectional shape and one or more conductive rings (e.g., contacts) spaced apart from each other at the distal end of the lead body. The conductive rings operate as individual electrodes and, in many cases, the SCS leads are implanted percutaneously through a needle inserted into the epidural space, with or without the assistance of a stylet. In other systems, the electrodes are carried by a paddle that is implanted via a laminotomy.

The signal generator transmits electrical pulses to the electrodes, creating an electric field, which in turn modifies the function of the patient's nervous system, such as by altering the patient's responsiveness to sensory stimuli and/or altering the patient's motor-circuit output. In SCS therapy for the treatment of pain, for example, the signal generator applies electrical pulses to the spinal cord via the electrodes. Document US 10 300 277 B1 discloses an implantable neurostimulator adapted to deliver a paresthesia-free electrical signal to a target neural population.

### BRIEF DESCRIPTION OF THE DRAWINGS

Figure 1 is a partially schematic illustration of an implantable spinal cord modulation system positioned at a patient's spine to deliver electrical signals in accordance with some embodiments of the present technology.
Figure 2A is a partially schematic, cross-sectional illustration of a patient's spine, illustrating representative locations for implanted lead bodies in accordance with some embodiments of the present technology.
Figure 2B is a partially schematic, cross-sectional illustration of a patient's brain, illustrating representative locations for implanted lead bodies in accordance with some embodiments of the present technology.
Figure 2C is a schematic illustration of a patient's peripheral nerve illustrating representative locations for implanted lead bodies in accordance with some embodiments of the present technology.
Figure 3 is a schematic illustration of a representative lead body suitable for providing modulation to a patient in accordance with some embodiments of the present technology.
Figure 4 is a flowchart of a method of treating a patient in accordance with some embodiments of the present technology.
Figure 5 is a flowchart of another method of treating a patient in accordance with some embodiments of the present technology.
Figure 6 is a table showing certain clinical data gathered from Applicant's clinical study on use of reduced stimulation schedules, in accordance with embodiments of the present technology.
Figures 7A-7F are graphs showing additional clinical data gathered from Applicant's clinical study on use of reduced stimulation schedules, in accordance with embodiments of the present technology.
Figures 8A and 8B are graphs showing additional clinical data gathered from Applicant's clinical study on use of reduced stimulation schedules, in accordance with embodiments of the present technology.

### DETAILED DESCRIPTION The invention is defined in claim 1.

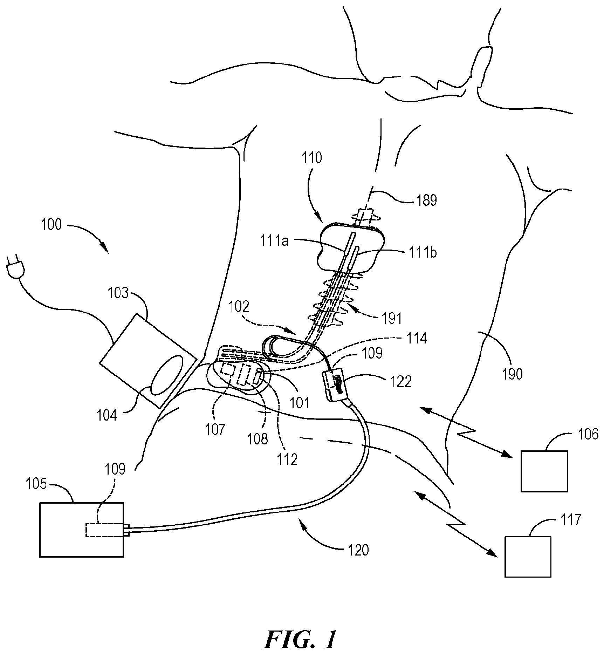

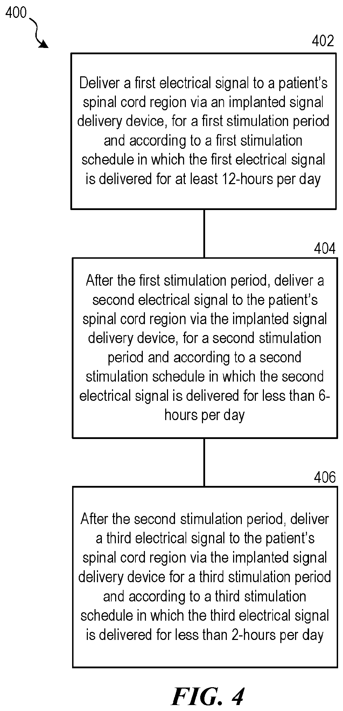

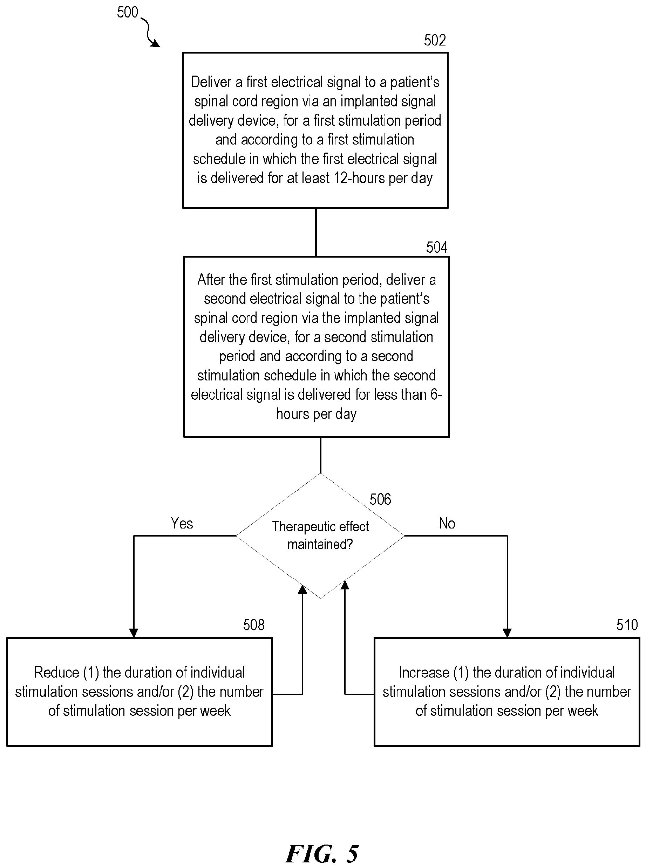

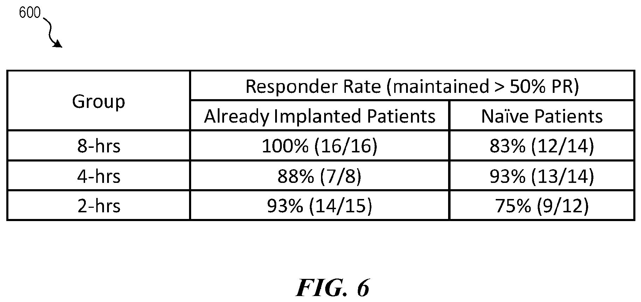

This Detailed Description includes the following headers and sections, which are provided for convenience only and do not affect the scope or meaning of the claimed present technology:
- Definitions of selected terms are provided under Heading 1.0 ("Definitions");
- General aspects of the present technology are described below under Heading 2.0 ("Overview of Present Technology");
- Representative treatment systems and their characteristics are described under Heading 3.0 ("System Characteristics") with reference to Figures 1-3;
- Representative embodiments of the present technology are described under Heading 4.0 ("Representative Embodiments of the Present Technology") with reference to Figures 4 and 5;
- Representative stimulation parameters of the present technology are described under Heading 5.0 ("Representative Stimulation Parameters");
- Representative clinical applications for the present technology are described under Heading 6.0 ("Representative Clinical Applications");
- Clinical data obtained in accordance with the present technology is described under Heading 7.0 ("Clinical Data") with reference to Figures 6-8B; and
- Representative examples are described under Heading 8.0 ("Representative Examples").

### 1.0 Definitions

Unless otherwise stated, the terms "generally," "about," and "approximately" refer to values within 10% of a stated value. For example, the use of the term "about 100" refers to a range of 90 to 110, inclusive. In instances in which relative terminology is used in reference to something that does not include a numerical value, the terms are given their ordinary meaning to one skilled in the art.

As used herein, and unless otherwise noted, the terms "modulate," "modulation," "stimulate," and "stimulation" refer generally to electrical signals that have an inhibitory, excitatory, and/or other effect on a target neural population. Accordingly, a spinal cord "stimulator" can have an inhibitory effect and/or an excitatory effect on certain neural populations. Moreover, the use of the terms "suppress" and "inhibit" in relation to an electrical signal's effect on a neuron refers to a reduction in the neuron's firing rate relative to the neuron's baseline firing rate in the absence of the electrical signal, and does not necessarily refer to a complete elimination of action potentials in the neuron. The "baseline" firing rate can refer to the neuron's spontaneous firing rate and/or the firing rate of the neuron in response to an external stimulus other than the therapy signal.

As used herein, the terms "neuromodulation signal", "electrical therapy signal," "electrical signal," "therapy signal," "signal," and other associated terms are used interchangeably and generally refer to an electrical signal that can be characterized by one more parameters, such as frequency, pulse width, and/or amplitude.

As used herein, the term "high frequency" when used to describe an electrical signal refers to an electrical signal having a frequency between 1.2 kHz and about 1 MHz, unless specifically stated otherwise. As used herein, the term "low frequency" when used to describe an electrical signal refers to an electrical signal having a frequency of less than 1.2 kHz, unless specifically stated otherwise.

As used herein, the term "paresthesia-free" when used to describe an electrical signal refers to an electrical signal that does not produce paresthesia when delivered to a patient. Paresthesia-free signals may have combinations of frequency, pulse widths, amplitudes, and/or pulse dosing rates that cause the signal to be below a patient's perception threshold. For example, paresthesia-free electrical signals may have a frequency of between about 1 Hz and about 1 MHz, or between about 1.2 kHz and 500 kHz, or between about 1.2 kHz and about 100 kHz. As another example, paresthesia-free electrical signals may be delivered in discrete bursts, separated by quiescent periods in which the electrical signal is not delivered. As yet another example, paresthesia-free electrical signals may have a "long" pulse width of between about 5 milliseconds and about 2 seconds with an amplitude below an activation threshold of a target neural population. Additional examples of paresthesia-free electrical signals, including those mentioned above, are described in U.S. Patent Application Publication Nos. US2010/0274314, US2021/0228881, and US2022/0401730, and U.S. Provisional Patent Application No. 63/501, 122.

As used herein, the term "pulse width" refers to the width of any phase of a repeating pulse, such as the portion of a pulse at a given polarity, unless explicitly described otherwise. For example, the use of the term pulse width with respect to a signal having bi-phasic pulses can refer to the duration of an anodic pulse phase or a cathodic pulse phase. The use of the term pulse width with respect to a signal having monophasic pulses can refer to the duration of the monophasic pulse phase.

As used herein, the term "pulse dosing" refers to repeatedly alternating between a first period in which pulses of an electrical signal are actively delivered and a second period in which the pulses are not delivered (e.g., a quiescent period). The pattern of alternating between the first (active) period and the second (quiescent) period is repeated for the "duration" the therapy is being applied for. The term "pulse dosing rate" or equivalents therefore refer to the percentage of the first period compared to the sum of the first period and the second period. For example, an electrical signal may be delivered continuously for 30 seconds (i.e., the first period equals 30 seconds), followed by a 30 second quiescent period (i.e., the second period equals 30 seconds). Such signal has a pulse dosing rate of 50%. In general, for purposes of pulse dosing, the second (quiescent) period is typically less than about 60 minutes, and more typically less than about 30 minutes, less than about 15 minutes, less than about 5 minutes, less than about 1 minute, or less than about 30 seconds. As described below, pulse dosing rates in accordance with the present technology can range from about 1% to about 100%.

In contrast, the terms "bolus" and "stimulation session" refer to a discrete period during which neurostimulation therapy is being applied to the patient, followed by a relatively prolonged off-period during which no neurostimulation therapy is applied to the patient. Of note, the neurostimulation therapy may be "pulsed dosed" during the discrete period during which neurostimulation therapy is being applied. For example, a "bolus" or "stimulation session" of one hour may include delivering an electrical stimulation at a pulse dose rate of 50%, such as alternating between 30 second active periods and 30 second quiescent periods for the one hour bolus period. In general, a bolus or stimulation session is typically followed by an off-period of greater duration than any quiescent period associated with pulse dosing. For example, a bolus or stimulation session is typically followed by an off-period of at least 1 hour, and more typically by an off-period of at least 2 hours, at least 3 hours, or at least 4 hours.

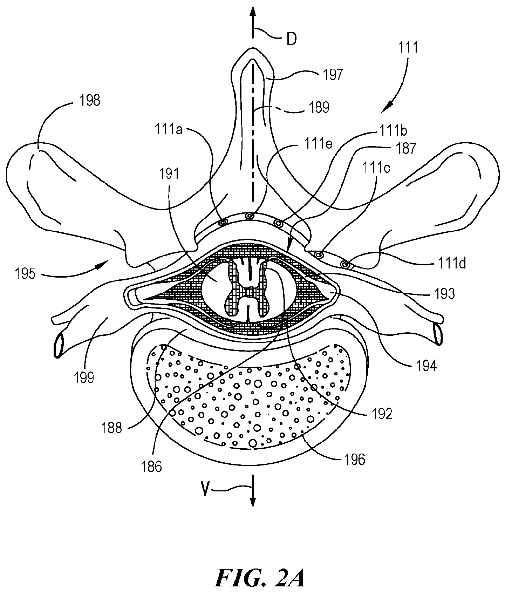

As used herein, "proximate a spinal cord region" refers to the placement of a signal delivery element or device such that it can deliver electrical stimulation to a neural population located in the spinal cord and/or within the spinal canal. For example, "proximate a spinal cord region" includes, but is not limited to, the relative lead positions described and shown in Figure 2A, as well as other positions not expressly described herein.

As used herein, "proximate a target neural population" refers to the placement of a signal delivery element such that it can deliver electrical stimulation to the target neural population. For example, if the target population includes neurons in the spinal cord at a given vertebral level, "proximate the target neural population" includes, but is not limited to, the relative lead positions described and shown in Figure 2A at the given vertebral level, as well as other positions not expressly described herein. As another example, if the target population includes neurons in the patient's cortex (e.g., motor cortex), "proximate the target neural population" includes, but is not limited to, leads positioned in or on the patient's cortex.

### 2.0 Overview of the Present Technology

Nevro Corp., the assignee of the present application, has previously developed and commercialized paresthesia-free spinal cord stimulation for treating pain and other conditions. For many patients, the paresthesia-free stimulation therapy provides superior pain relief and/or other symptom alleviation compared to conventional paresthesia-based stimulation. The paresthesia-free stimulation also often provides fewer side effects compared to conventional paresthesia-based stimulation.

However, one drawback of some paresthesia-free therapies is that more energy/power is required to generate and deliver the therapy. For example, more energy/power is generally required to generate and deliver a high frequency, paresthesia-free electrical signal than a low frequency, paresthesia-based electrical signal for the same duration. This is because, for a single pulse cycle consisting of a pulse and an interpulse interval, a high frequency signal typically has a higher percent "on" time than a low frequency signal. That is, the ratio between the pulse width and the total duration of the pulse cycle is generally higher for a high frequency signal. This means that the amount of time a system must provide power (e.g., the sum of all the pulse widths over a given period) is higher for high frequency stimulation than low frequency stimulation. As a result, high frequency, paresthesia-free stimulation systems may require larger batteries (and thus larger implants) and/or may require the patient to recharge the system more frequently. Despite these inconveniences, paresthesia-free therapy is still preferred by many patients over paresthesia-based therapy due to its superior effectiveness and reduced side effects.

The present technology provides systems and methods that are expected to at least partially address these inconveniences associated with the higher power requirement of some paresthesia-free neurostimulation therapies, such as high frequency, paresthesia-free therapy. In particular, the present technology provides stimulation schedules/regimens that are expected to reduce the amount of power required for extended paresthesia-free stimulation therapy, while maintaining the benefits of paresthesia-free stimulation. As described below, this is accomplished by reducing the dose of paresthesia-free neurostimulation provided to a patient over an extended time. Of note, the stimulation dose can be reduced in accordance with the therapy regimens described herein without a corresponding reduction, or meaningful reduction, in effectiveness. For example, paresthesia-free stimulation can induce a prolonged "wash out" period during which symptomatic relief (e.g., pain relief) persists even after delivery of the electrical signal is stopped. Accordingly, stimulation can be periodically and repeatedly turned off without a corresponding reduction, or meaningful reduction, in effectiveness. This is in contrast with paresthesia-based therapies, which require paresthesia (and thus continuous application of the signal to induce paresthesia) to maintain pain relief. Reducing the dose of paresthesia-free neurostimulation provided to a patient over time may facilitate additional patient conveniences beyond reducing power-consumption, such as enabling smaller implants, enabling externally powered devices, negating the need for an implanted signal generator and/or an implantable rechargeable battery, enabling using a primary cell battery in the implant, among others.

Specific details of certain embodiments of the disclosure are described below with reference to methods for modulating one or more target neural populations (e.g., nerves) or sites of a patient, and associated implantable structures for providing the modulation. Although selected embodiments are described below with reference to modulating the dorsal column, dorsal horn, dorsal root, dorsal root entry zone, ventral column, ventral horn, and/or other particular regions of the spinal column, the modulation may in some instances be directed to other neurological structures and/or target neural populations of the spinal cord and/or other neurological tissues throughout the body. For example, some embodiments may include modulating brain tissue, including the cortex (e.g., motor cortex) and/or deep brain structures. As another example, some embodiments may include modulating peripheral nervous tissue, such as dorsal root ganglion, sacral nerves, the vagal nerve, the median nerve, the tibial nerve, or other peripheral nerves. Some embodiments can have configurations, components, or procedures different than those described in this section, and other embodiments may eliminate particular components or procedures. A person of ordinary skill in the relevant art, therefore, will understand that the present disclosure may include other embodiments with additional elements, and/or may include other embodiments without several of the features shown and described below with reference to Figures 1-5.

### 3.0 System Characteristics

Figure 1 schematically illustrates a representative patient therapy system 100 for treating a patient's motor, sensory, and/or other functioning, arranged relative to the general anatomy of the spinal column 191 of a patient 190. The system 100 can include a signal generator 101 (e.g., an implanted or implantable pulse generator or IPG), which can be implanted subcutaneously within a patient 190 and coupled to one or more signal delivery elements or devices 110. The signal delivery elements or devices 110 can be implanted within the patient 190, at or off the patient's spinal cord midline 189. The signal delivery elements 110 carry features for delivering therapy to the patient 190 after implantation. The signal generator 101 can be connected directly to the signal delivery devices 110, or it can be coupled to the signal delivery devices 110 via a signal link, e.g., a lead extension 102. In some embodiments, the signal delivery devices 110 can include one or more elongated lead(s) or lead body or bodies 111 (identified individually as a first lead 111a and a second lead 111b). As used herein, the terms signal delivery device, signal delivery element, lead, and/or lead body include any of a number of suitable substrates and/or supporting members that carry electrodes/devices for providing therapy signals to the patient 190. For example, the lead or leads 111 can include one or more electrodes or electrical contacts that deliver electrical signals into the patient's tissue, e.g., to provide for therapeutic relief. In some embodiments, the signal delivery elements 110 can include structures other than a lead body (e.g., a paddle) that also deliver electrical signals and/or other types of signals to the patient 190, e.g., as disclosed in U.S. Patent Application Publication No. 2018/0256892. In some embodiments, paddles can be more suitable for patients with stenosis or other indications that compromise the epidural space and preclude the percutaneous deliver of cylindrical leads.

In some embodiments, one signal delivery device can be implanted on one side of the spinal cord midline 189, and a second signal delivery device can be implanted on the other side of the spinal cord midline 189. For example, the first and second leads 111a, 111b shown in Figure 1 can be positioned just off the spinal cord midline 189 (e.g., about 1 mm offset) in opposing lateral directions so that the two leads 111a, 111b are spaced apart from each other by about 2 mm. In some embodiments, the leads 111 can be implanted at a vertebral level ranging from, for example, about T1 to about T12, or from about T4 to about T12. In some embodiments, one or more signal delivery devices can be implanted at other vertebral levels, e.g., as disclosed in U.S. Patent No. 9,327. 121. In other embodiments, one or more leads 111 can be implanted at or proximate other target neural structures, including brain tissue, peripheral nerves, etc.

The signal generator 101 can transmit signals (e.g., electrical signals) to the signal delivery elements 110 that excite, inhibit, downregulate and/or suppress target nerves. The signal generator 101 can include a machine-readable (e.g., computer-readable or controller-readable) medium containing instructions for generating and transmitting suitable therapy signals, such as to perform the methods described below with respect to Figures 4 and 5. The signal generator 101 and/or other elements of the system 100 can include one or more processor(s) 107, memory unit(s) 108, and/or input/output device(s) 112. Accordingly, the process of providing modulation signals, providing guidance information for positioning the signal delivery devices 110, establishing battery charging and/or discharging parameters, and/or executing other associated functions can be performed by computer-executable instructions contained by, on, or in computer-readable media located at the pulse generator 101 and/or other system components. Further, the pulse generator 101 and/or other system components can include dedicated hardware, firmware, and/or software for executing computer-executable instructions that, when executed, perform any one or more methods, processes, and/or sub-processes described herein and/or in the materials. The dedicated hardware, firmware, and/or software also serve as "means for" performing the methods, processes, and/or sub-processes described herein. The signal generator 101 can also include multiple portions, elements, and/or subsystems (e.g., for directing signals in accordance with multiple signal delivery parameters), carried in a single housing, as shown in Figure 1, or in multiple housings. For example, the signal generator can include some components that are implanted (e.g., a circuit that directs signals to the signal delivery device 110), and some that are not (e.g., a power source). The computer-executable instructions can be contained on one or more media that are implanted within the patient and/or positioned external to the patient, depending on the embodiment.

The signal generator 101 can also receive and respond to an input signal received from one or more sources. The input signals can direct or influence the manner in which the therapy, charging, and/or process instructions are selected, executed, updated, and/or otherwise performed. The input signals can be received from one or more sensors (e.g., an input device 112 shown schematically in Figure 1 for purposes of illustration) that are carried by the signal generator 101 and/or distributed outside the signal generator 101 (e.g., at other patient locations) while still communicating with the signal generator 101. The sensors and/or other input devices 112 can provide inputs that depend on or reflect patient state (e.g., patient position, patient posture, and/or patient activity level), and/or inputs that are patient-independent (e.g., time). Still further details are included in U.S. Patent No. 8, 355, 797.

In some embodiments, the signal generator 101 and/or signal delivery devices 110 can obtain power to generate the therapy signals from an external power source 103. In some embodiments, the external power source 103 can bypass an implanted signal generator and generate a therapy signal directly at the signal delivery devices 110 (or via signal relay components). The external power source 103 can transmit power to the implanted signal generator 101 and/or directly to the signal delivery devices 110 using electromagnetic induction (e.g., RF signals). For example, the external power source 103 can include an external coil 104 that communicates with a corresponding internal coil (not shown) within the implantable signal generator 101, signal delivery devices 110, and/or a power relay component (not shown). In some embodiments, the external power source 103 can transmit power to the implanted signal generator 101 and/or directly to the signal delivery devices 110 in a generally continuous manner such that the system 100 can operate without an internal power source. The external power source 103 can be portable for ease of use.

In some embodiments, the implanted signal generator 101 can be omitted and the external power source 130 can be configured as an external signal generator that transmits power and/or electrical signals to the signal delivery devices 110 (e.g., via an implanted relay device; not shown). For example, the external power source 103 can either transmit the electrical signal itself to the signal delivery device or cause an electrical signal to be generated directly at the signal delivery devices 110 or at an implanted relay device (not shown). In such embodiments, the external power source 103 can be a wearable device that the patient wears while receiving therapy. In such embodiments, the patient only receives stimulation therapy while the wearable device is placed in an active state and is being worn by the patient. This is generally less invasive because such embodiments generally do not require an implanted signal generator 101, nor an implanted power storage device. In such embodiments, the external power source 103 may be wireless to enable patient mobility and/or accurate positioning of the external power source 103 during treatment. For example, the external power source 103 can include a rechargeable battery (not shown) that can be used to power the external power source 103 while in the active state, and recharged while in an inactive state.

In some embodiments, the signal generator 101 can obtain the power to generate therapy signals from an internal power source, in addition to or in lieu of the external power source 103. For example, the implanted signal generator 101 can include a non-rechargeable battery (e.g., a primary cell) or a rechargeable battery (e.g., a secondary cell) to provide such power. When the internal power source includes a rechargeable battery, the external power source 103 can be used to recharge the battery. The external power source 103 can in turn be recharged from a suitable power source (e.g., conventional wall power).

During at least some procedures, an external stimulator or trial modulator 105 can be coupled to the signal delivery elements 110, e.g., during an initial procedure, prior to implanting the signal generator 101. For example, a practitioner (e.g., a physician and/or a company representative) can use the trial modulator 105 to vary the modulation parameters provided to the signal delivery elements 110 in real time, and select optimal or particularly effective parameters. These parameters can include the location from which the electrical signals are emitted, as well as the characteristics of the electrical signals provided to the signal delivery devices 110. In some embodiments, input is collected via the external stimulator or trial modulator 105 and can be used by the clinician to help determine what parameters to vary. In a typical process, the practitioner uses a wireless connection or cable assembly 120 to temporarily connect the trial modulator 105 to the signal delivery device 110. The practitioner can test the effectiveness of the signal delivery devices 110 in an initial position. The practitioner can then disconnect the cable assembly 120 if needed (e.g., at a connector 122), reposition the signal delivery devices 110, and reapply the electrical signals. This process can be performed iteratively until the practitioner obtains the desired position for the signal delivery devices 110. Optionally, the practitioner can move the partially implanted signal delivery devices 110 without disconnecting the cable assembly 120. Furthermore, in some embodiments, the iterative process of repositioning the signal delivery devices 110 and/or varying the therapy parameters may not be performed.

The signal generator 101, the lead extension 102, the trial modulator 105 and/or the connector 122 can each include a receiving element 109. Accordingly, the receiving elements 109 can be patient implantable elements, or the receiving elements 109 can be integral with an external patient treatment element, device or component (e.g., the trial modulator 105 and/or the connector 122). The receiving elements 109 can be configured to facilitate a simple coupling and decoupling procedure between the signal delivery devices 110, the lead extension 102, the pulse generator 101, the trial modulator 105 and/or the connector 122. The receiving elements 109 can be at least generally similar in structure and function to those described in U.S. Patent Application Publication No. 2011/0071593.

After the signal delivery elements 110 are implanted, the patient 190 can receive therapy via signals generated by the trial modulator 105 or via another external signal generator (e.g., the power source 103), generally for a limited period of time. During this time, the patient wears the trial modulator 105 outside the body. Assuming the trial therapy is effective or shows the promise of being effective, the practitioner then replaces the trial modulator 105 with the implanted signal generator 101, and programs the signal generator 101 with therapy programs selected based on the experience gained during the trial period. Optionally, the practitioner can also replace the signal delivery elements 110. In still further embodiments, the signal generator 101 can be implanted without first undergoing a trial period. Once the implantable signal generator 101 has been positioned within the patient 190, the therapy programs provided by the signal generator 101 can still be updated remotely via a wireless physician's programmer 117 (e.g., a physician's laptop, a physician's remote or remote device, etc.) and/or a wireless patient programmer 106 (e.g., a patient's laptop, patient's remote or remote device, etc.). Generally, the patient 190 has control over fewer parameters than does the practitioner. For example, the capability of the patient programmer 106 can be limited to starting and/or stopping the signal generator 101, selecting a pre-programmed therapy option, and/or adjusting the signal amplitude within a present amplitude range. The patient programmer 106 can be configured to accept inputs corresponding to pain relief, motor functioning and/or other variables, such as medication use. Accordingly, more generally, embodiments of the present technology include receiving patient feedback, via a sensor, that is indicative of, or otherwise corresponds to, the patient's response to the signal. Feedback includes, but is not limited to, motor, sensory, and verbal feedback. In response to the patient feedback, one or more signal parameters can be adjusted, such as frequency, pulse width, amplitude, or delivery location. In some embodiments, the patient programmer can be a network connected handheld computing device such as a smartphone, which can include a patient app that provides various functions such as remote programming, therapy selection, therapy tracking information such as current pain score or level of sensory restoration for use by the clinician and/or software in the app to optimize therapy.

Figure 2A is a cross-sectional illustration of the spinal cord 191 and an adjacent vertebra 195 (based generally on information from Crossman and Neary, "Neuroanatomy," 1995 (published by Churchill Livingstone)), along with multiple leads 111 (shown as leads 111a-111e) implanted at representative locations. For purposes of illustration, multiple leads 111 are shown in Figure 2A implanted in a single patient. In addition, for purposes of illustration, the leads 111 are shown as elongated leads however, leads 111 can be paddle leads. In actual use, any given patient will likely receive fewer than all the leads 111 shown in Figure 2A.

The spinal cord 191 is situated within a vertebral foramen 188, between a ventrally located ventral body 196 and a dorsally located transverse process 198 and spinous process 197. Arrows V and D identify the ventral and dorsal directions, respectively. The spinal cord 191 itself is located within the dura mater 199, which also surrounds portions of the nerves exiting the spinal cord 191, including the ventral roots 192, dorsal roots 193, and dorsal root ganglia 194. The dorsal roots 193 enter the spinal cord 191 at the dorsal root entry region 187, and communicate with dorsal horn neurons located at the dorsal horn 186. In some embodiments, the first and second leads 111a, 111b are positioned just off the spinal cord midline 189 (e.g., about 1 mm offset) in opposing lateral directions so that the two leads 111a, 111b are spaced apart from each other by about 2 mm, as discussed above. In some embodiments, a lead or pairs of leads can be positioned at other locations, e.g., toward the outer edge of the dorsal root entry region 187 as shown by a third lead 111c, or at the dorsal root ganglia 194, as shown by a fourth lead 111d, or approximately at the spinal cord midline 189, as shown by a fifth lead 111e. One or more of the leads 111a-11e can deliver neurostimulation to various targets within the spinal cord spinal cord, including, but not limited to, the dorsal column, the dorsal horn, the intermediolateral nucleus, and/or other neurons within any of laminae I-X of the spinal cord.

In some embodiments, the devices and systems of the present technology include features other than those shown herein. For example, one lead 111 to six leads 111 can be positioned generally end-to-end at or near the patient's midline M and span vertebral levels from about C2 to about T12, or from about T4 to about T12. In some embodiments, two, three, or four leads 111 are positioned end-to-end at or near the patient's midline from T4 to T12. In some embodiments, the leads 111 and/or other signal delivery devices can have locations other than those expressly shown herein. For example, one or more signal delivery devices can be positioned at the dorsal side of the spinal cord 191. In addition, the devices and systems of the present technology can include more than one internal stimulator and/or more than one external stimulator that can be configured for wireless stimulation, such as by using electromagnetic waves.

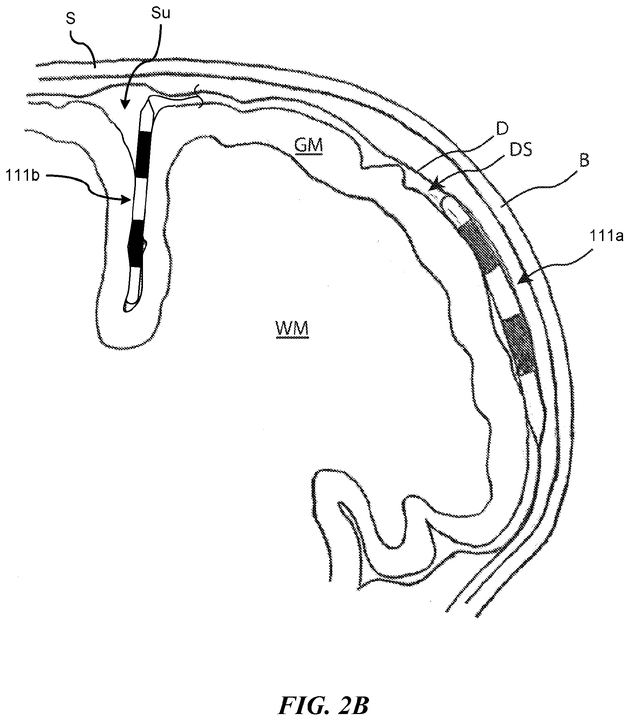

In some embodiments, the signal delivery elements 110 can be positioned within the patient's head to modulate neurons at any suitable lobe or other structure of the cortex or deep brain. For example, Figure 2B is a partially schematic, cross-sectional illustration of a first lead 111a placed at a first cortical location and a second lead 111b placed at a second cortical location. The first lead 111a is shown as positioned within the patient's dura D, in the subdural space DS, so as to lie along or adjacent to the gray matter GM of the patient's cortex. The second lead 111b is shown as positioned within a sulcus S (e.g., the central sulcus Su) so as to better access neural populations within the folds of the patient's cortex. Of course, the signal delivery element(s) (e.g., leads) can be positioned at other cortical locations (e.g., epidurally). In some embodiments, the signal delivery elements 110 can be positioned within or proximate deep brain regions, in addition to or in lieu of placement at cortical regions. Representative deep brain targets include, but are not limited to, the subthalamic nucleus (STN), the globus pallidus internus (GPI), the ventral intermediate (VIM) nucleus, the pedunculopontine nucleus (PPN), the hippocampus, the amygdala, and the like.

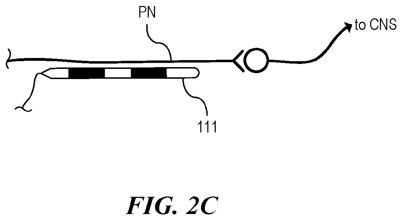

In some embodiments, the signal delivery elements 110 can be positioned at or proximate one or more peripheral nerves of the patient to modulate peripheral neurons. For example, Figure 2C is a schematic representation of a lead 111 positioned adjacent a peripheral nerve PN. In some embodiments, the peripheral nerve PN includes a bundle of nerve fibers. Representative peripheral nerves include, but are not limited to, the vagal nerve, sacral nerves, median nerves, tibial nerves, femoral nerves, sciatic nerve, obturator nerve, pudendal nerve, saphenous nerve, fibular nerve, sural nerve, axillary nerve, radial nerve, ulnar nerve, musculocutaneous nerve, etc. The lead 111 can be placed at various positions along the peripheral nerve PN, including adjacent the nerve fibers (e.g., along the length of the axons), adjacent nerve bodies, or adjacent both nerve fibers and nerve bodies. Although shown as a cylindrical lead, in some embodiments the lead 111 can be a cuff electrode configured to at least partially wrap around the peripheral nerve PN.

Several aspects of the technology are embodied in computing devices, e.g., programmed/programmable pulse generators, controllers and/or other devices. The computing devices on/in which the described technology can be implemented can include one or more central processing units, memory, input devices (e.g., input ports), output devices (e.g., display devices), storage devices, and network devices (e.g., network interfaces). The memory and storage devices are computer-readable media that can store instructions that implement the technology. In some embodiments, the computer readable media are tangible media. In some embodiments, the data structures and message structures can be stored or transmitted via an intangible data transmission medium, such as a signal on a communications link. Various suitable communications links can be used, including but not limited to a local area network and/or a wide-area network.

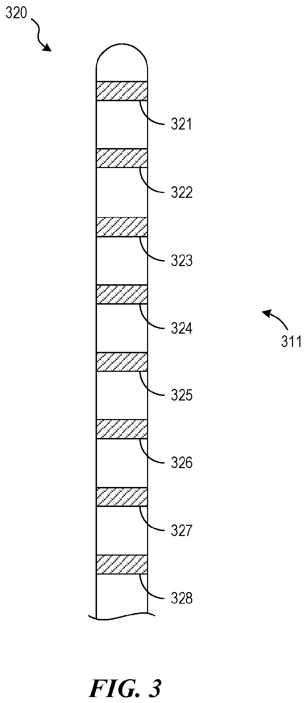

Figure 3 is a partially schematic illustration of a representative lead body 311 that can be used to apply modulation to a patient in accordance with any of the foregoing embodiments. In general, the lead body 311 includes a multitude of electrodes or contacts 320. When the lead body 311 has a circular cross-sectional shape, as shown in Figure 3, the contacts 320 can have a generally ring-type or segmented shape and can be spaced apart axially along the length of the lead body 311. In a particular embodiment, the lead body 311 can include eight contacts 320, identified individually as first, second, third . . . eighth contacts 321, 322, 323 . . . 328, although in other embodiments the lead body 311 can include fewer or more electrodes, such as between 1 electrode and 64 electrodes. In general, one or more of the contacts 320 are used to provide signals, and another one or more of the contacts 320 provide a signal return path. Accordingly, the lead body 311 can be used to deliver monopolar modulation (e.g., if the return contact is spaced apart significantly from the delivery contact), or bipolar modulation (e.g., if the return contact is positioned close to the delivery contact and in particular, at the same target neural population as the delivery contact). In still further embodiments, the pulse generator 101 (Figure 1) can operate as a return contact for monopolar modulation.

### 4.0 Representative Embodiments of the Present Technology

As set forth above, the present technology includes stimulation schedules/regimens that are expected to reduce the amount of power required for paresthesia-free stimulation, while maintaining the benefits of paresthesia-free stimulation. Additionally, the present technology includes stimulation schedules/regimens that are expected to reduce the burden on a patient undergoing stimulation in circumstances in which an external power source is required to be worn during the therapy stimulation. This is accomplished by reducing the dose of paresthesia-free neurostimulation provided to a patient over time. Of note, the reduction in dose can occur without a corresponding reduction, or clinically meaningful reduction, in effectiveness.

In some embodiments, the patient may initially receive paresthesia-free stimulation on a continuous or generally continuous basis for a first stimulation period, such as immediately following implantation of the signal delivery device. As used herein, the term "continuous" refers to administering an electrical signal 24 hours per day without any significant off-periods (e.g., without an "off-period" of one or more consecutive hours). The signal may be pulse dosed for the 24 hour period and still be considered a "continuous" signal. As described above, suitable pulse dose rates include between 1% and 100%. As also described above, pulse dose patterns frequently operate on a timescale of minutes or less (e.g., 1 minute on, 1 minute off, 1 second on, 1 second off; 100 milliseconds on, 1 second off; etc.). Accordingly, the term continuous does not preclude a signal being delivered accordingly to a pulse dose, but rather is used to contrast other stimulation schedules described below, in which stimulation is ceased for a consecutive off-period on the order of hours (e.g., the signal is off for greater than at least 1 consecutive hour).

In some embodiments, the patient may initially receive paresthesia-free stimulation on a noncontinuous basis for the first stimulation period. In such embodiments, the patient nevertheless receives a relatively significant dose of stimulation during the first stimulation period, such as at least 12 hours of stimulation per day. The at least 12 hours of stimulation can be consecutive/uninterrupted (e.g., 12 hours on, 12 hours off), or nonconsecutive (e.g., 4 hours on, 4 hours off, repeated over the course of 24 hours). As described above, the signal may be administered according to a pulse dose during the "on" time (e.g., the signal has a pulse dose of 50% for the 12 hour "on" period and is ceased entirely during the 12 hour "off" period).

The first stimulation period can be between about 1 month and about 1 year, such as between about 1 month and about 9 months, between about 1 month and about 6 months, and/or between about 1 month and about 3 months. For example, the first period can be about 1 month, about 2 months, about 3 months, about 4 months, about 5 months, or about 6 months. The first period may also be shorter. For example, in some embodiments the first period may be between about 1 day and about 30 days, such as between about 10 days and about 30 days, or about 10 days, about 15 days, about 20 days, about 25 days, or about 30 days.

After receiving continuous or generally continuous paresthesia-free stimulation for the first stimulation period, the patient may then receive a reduced dose of paresthesia-free stimulation during a second stimulation period that follows the first stimulation period. The reduced dose of stimulation can be noncontinuous (e.g., intermittent) stimulation in which stimulation is only administered during discrete therapeutic or stimulation sessions. Such discrete stimulation sessions may also be referred to herein as a "bolus" of stimulation. As described below, stimulation schedules during the second stimulation period generally include an "off-period" of at least 12 hours. In many embodiments described below, the off-period is greater than 12 hours, such as at least 18 hours or more.

In some embodiments, delivering the reduced dose of stimulation includes delivering stimulation during a single stimulation session per day (e.g., a once per day bolus). The once per day stimulation session may include a consecutive (e.g., uninterrupted) duration of between about 1 hour and about 12 hours, such as between about 1 hour and about 9 hours, or between about 1 hour and about 6 hours, or between about 1 hour and about 4 hours, or between about 2 hours and about 4 hours, or between about 1 hour and about 3 hours, or between about 1 hour and about 2 hours. For example, the stimulation session may have a duration of about 1 hour, about 2 hours, about 3 hours, about 4 hours, about 5 hours, about 6 hours, about 7 hours, or about 8 hours. The once per day stimulation session may also have shorter durations. For example, the stimulation session may have a duration of between about 1 minute and about 60 minutes, such as between about 5 minutes and about 60 minutes, or between about 15 minutes and about 45 minutes. Representative examples include about 2 minutes, about 5 minutes, about 10 minutes, about 15 minutes, about 20 minutes, about 25 minutes, about 30 minutes, about 35 minutes, about 40 minutes, about 45 minutes, about 50 minutes, and about 55 minutes. As described above with respect to continuous stimulation, stimulation can be administered during the stimulation session according to a pulse dose. The patient does not receive clinically significant stimulation other than during the stimulation session.

In some embodiments, delivering the reduced dose of stimulation includes delivering stimulation during two, three, four, or more stimulation sessions per day (e.g., a twice per day bolus, three times per day bolus, etc.). Each stimulation session may have a duration equal to any of the durations recited above for the once per day stimulation session. However, in embodiments in which multiple stimulation sessions occur each day, the duration of each stimulation session is generally less than about 2 hours, such as between about 1 minute and about 1 hour, or about 5 minutes, about 10 minutes, about 15 minutes, about 30 minutes, about 45 minutes, or about 1 hour. Moreover, each stimulation session need not be the same length. For example, a first stimulation session may be about 45 minutes, and a second stimulation session may be about 15 minutes, or vice versa. In embodiments in which more than one stimulation session occurs per day, the stimulation sessions are generally spaced apart by at least one hour, at least two hours, at least three hours, or more, during which the patient receives no stimulation.

In some embodiments, delivering the reduced dose of stimulation includes delivering stimulation during stimulation sessions that occur less frequently than once per day. For example, the reduced dose may include one stimulation session every other day, one stimulation session every third day, one stimulation session per week, etc. Similarly, the reduced dose may include a number of stimulation session spread over a number of days, such as two stimulation session over a five-day period, three stimulation sessions over a five-day period, four stimulation sessions over a seven-day period, or the like.

In some embodiments, the duration of the first stimulation period is predetermined such that the patient transitions to the reduced dose after the predetermined duration. In some embodiments, the predetermined duration can be based at least in part on durations associated with successful outcomes in reference patient data (e.g., aggregated patient data collected during clinical studies). Once the predetermined duration has elapsed, the stimulation system can automatically deliver, or be reprogrammed to deliver, the reduced dose of stimulation. In other embodiments, the transition between the first stimulation period and the second stimulation period is not automatic. For example, in embodiments in which the signal generator is a wearable device, the patient may simply be instructed after the predetermined duration of the first stimulation period to reduce their usage of the stimulation system. In such embodiments, the healthcare provider can "prescribe" the patient a specific number and duration of stimulation sessions for the second stimulation period (e.g., two 30-minute stimulation session per day; four one-hour stimulation sessions per week; etc.), similar to a healthcare provider instructing a patient to attend physical therapy a set number of days per week. By way of one example, this "prescription" can take place via remote programming of the patient smartphone application or other patient controller described herein.

In some embodiments, the duration of the first stimulation period is based on one or more measured or reported criteria meeting certain predetermined thresholds. Suitable objective criteria include, for example, spontaneous neural activity, evoked neural activity, patient activity level, etc. Suitable subjective criteria include, for example, patient-reported pain scores (e.g., VAS pain scores, NRS scores, etc.), patient-reported quality of life, patient satisfaction, patient sleep quality, physician observations, etc. Once the one or more objective or subjective criteria meet the predetermined threshold, the signal generator can automatically transition to delivering the reduced dose of stimulation. In other embodiments, the transition is not automatic. For example, in embodiments in which the signal generator is a wearable device, the patient may simply be instructed (e.g., by their healthcare provider, by a patient-controller, etc.) to reduce their usage of the stimulation system once the objective or subjective criteria meet the predetermined threshold. In such embodiments, and as described above, the healthcare provider can "prescribe" the patient a specific number and duration of stimulation sessions for the second stimulation period. In some embodiments, the objective/subjective criteria can continue to be periodically monitored during the second stimulation period to determine whether the "prescription" (i.e., the amount and duration of stimulation sessions) should be adjusted.

In some embodiments, the second stimulation period continues for an indefinite period. For example, once the patient has transitioned to receiving the reduced dose of stimulation during the second stimulation period, the patient may continue to receive stimulation according to the reduced schedule indefinitely. Of course, the patient can be periodically reevaluated to determine whether the reduced dose needs to be adjusted, and/or whether the patient would benefit from returning to continuous or generally continuous stimulation. Similarly, as described above, subjective and objective criteria can continue to be collected to determine whether the "prescription" should be adjusted.

In some embodiments, the second stimulation period has a predetermined duration, after which the patient receives a second reduced dose of stimulation during a third stimulation period. The second reduced dose can be less than the initial reduced dose delivered during the second stimulation period. For example, if the initial reduced dose delivered during the second stimulation period is 2 hours per day, the second reduced dose during the third stimulation period can be 1 hour per day. As another example, if the initial reduced dose delivered during the second stimulation period is 1 hour per day, the second reduced dose during the third stimulation period can be 30 minutes per day. Of course, the foregoing are provided by way of example only-the second reduced dose can have any value within the ranges previously described with respect to the first reduced dose. In embodiments in which the patient transitions to a second reduced dose, the transition between the second stimulation period and the third stimulation period can be based on the same criteria as transitioning between the first stimulation period and the second stimulation period. In some embodiments, the second reduced dose can be greater than the first reduced dose, e.g., if deemed appropriate by a clinician or other healthcare provider to meet a therapy objective. For example, if the initial reduced dose during the second stimulation period is 2 hours per day, the second reduced dose during the third stimulation period may be increased to 4 hours per day.

In some embodiments, the patient can further be titrated through additional reduced doses during subsequent stimulation periods. For example, a patient may be titrated through three, four, five, six, seven, eight, or more stimulation periods each having a reduced (or at least different) dose of stimulation. For example, a patient may receive 12 hours of stimulation per day during the first stimulation period, 8 hours of stimulation per day during the second stimulation period, 4 hours of stimulation per day during the third stimulation period, 2 hours of stimulation per day during a fourth stimulation period, and 30 minutes of stimulation per day during a fifth stimulation period. Additional representative schedules for titration include any combination of the following: 8 hours, 4 hours, 2 hours, 1 hour, or 30 minutes of stimulation per day for the second stimulation period; 4 hours, 2 hours, 1 hour, 30 minutes, 15 minutes, or 5 minutes of stimulation per day for the third stimulation period; and 2 hours, 1 hour, 30 minutes,15 minutes, 5 minutes, or 1 minute of stimulation per day for the fourth stimulation period.

Without being bound by theory, applying stimulation on a continuous or generally continuous basis for a first stimulation period (e.g., immediately following implantation of the signal delivery device) is expected to induce a physiologic effect, that in turn is expected to produce a therapeutic effect. The first physiologic effect may be, for example, a quieting or other normalization of overactive or sensitized neurons, and the therapeutic effect may be a reduction in patient pain. Without being bound by theory, it is expected that less frequent stimulation (e.g., a lower dose of stimulation) is necessary to maintain the physiologic effect (and thus the therapeutic effect) once it has been induced. That is, the physiologic effect can be maintained by providing a reduced dose of stimulation, such as by providing stimulation on an intermittent basis as described above with respect to the second stimulation period. It is therefore expected that continuous or generally continuous stimulation is not necessary to maintain the physiologic effect once it has been achieved. Of course, response to stimulation varies by patient, so certain patients may require more stimulation to achieve and/or maintain the physiologic effect than others.

Although the foregoing describes administering continuous (or generally continuous) stimulation for a first stimulation period and a reduced dose of stimulation during a second stimulation period, in some embodiments the patient may begin therapy on an intermittent or noncontinuous stimulation schedule. For example, the patient may initially receive a first reduced dose of stimulation, such as described above with respect to the second stimulation period, before subsequently transitioning to a second reduced dose of stimulation, such as described above with respect to the third stimulation period. In some embodiments, therefore, the patient bypasses the first stimulation period and simply begins therapy at the second stimulation period. In such examples, the patient may begin therapy by receiving less than 12 hours of stimulation per day, such as about 8 hours of stimulation per day, 4 hours of stimulation per day, 2 hours of stimulation per day, 30 minutes of stimulation per day, or any of the other values described above with reference to the second stimulation period.

Figure 4 is a block diagram illustrating a method 400 for treating a patient in accordance with embodiments of the present technology. Some or all of the operations in the method 400 can be performed by a processor executing instructions stored on one or more elements of a patient treatment system, including the patient treatment system 100 described with reference to Figure 1.

The method 400 can begin at block 402 by delivering a first electrical signal to the patient's spinal cord region via an implanted signal delivery device, for a first stimulation period and according to a first stimulation schedule in which the first electrical signal is delivered for at least 12 hours per day. In some embodiments, delivering the first electrical signal according to the first stimulation schedule includes continuously delivering the first electrical signal during the first stimulation period, as described above. In other embodiments, delivering the first electrical signal according to the first stimulation schedule includes non-continuously delivering the first electrical signal during the first stimulation period. However, in embodiments in which the first electrical signal is delivered non-continuously, the first electrical signal is nevertheless delivered for at least 12 hours per day, such as at least 16 hours per day, or at least 20 hours per day. The first stimulation period can have any of the durations described above.

The method 400 can continue at block 404 by delivering, after the first stimulation period, a second electrical signal to the patient's spinal cord region via the implanted signal delivery device, for a second stimulation period and according to a second stimulation schedule in which the second electrical signal is delivered for less than 6 hours per day, such as less than 5 hours per day, or less than 4 hours per day, etc.. This may include, for example, delivering the second electrical signal during one or more stimulation sessions per day. The stimulation session can have a duration between about 1 minute and about 6 hours, as previously described. In some embodiments, the second electrical signal is delivered at block 404 during a single stimulation session per day, although in other embodiments the second electrical signal is delivered at block 404 during multiple stimulation sessions per day. In yet other embodiments, the second electrical signal is delivered during fewer than seven stimulation sessions per week such that the patient averages less than one stimulation session per day.

In some embodiments, the second stimulation period continues indefinitely. In other embodiments, the method 400 can optionally continue at block 406 by delivering, after the second stimulation period, a third electrical signal to the patient's spinal cord region via the implanted signal delivery device for a third stimulation period and according to a third stimulation schedule in which the third electrical signal is delivered for less than 2 hours per day, such as less than 1 hour per day, or less than 30 minutes per day. This may include, for example, delivering the third electrical signal during one or more stimulation sessions per day. The stimulation session can have a duration between about 1 minutes and about 2 hours. In some embodiments, the third electrical signal is delivered at block 406 during a single stimulation session per day, although in other embodiments the third electrical signal is delivered at block 404 during multiple stimulation sessions per day. In yet other embodiments, the third electrical signal is delivered during fewer than seven stimulation sessions per week such that the patient averages less than one stimulation session per day.

Although not shown in Figure 4, the method 400 can optionally continue by titrating the patient through additional stimulation periods having different stimulation schedules after the third stimulation period. For example, after the third stimulation period, the method 400 can include delivering a fourth electrical signal to the patient's spinal cord region for a fourth stimulation period and according to a fourth stimulation schedule in which the fourth electrical signal is delivered for less than, e.g., 1 hour per day. After the fourth stimulation period, the method 400 can further include delivering a fifth electrical signal to the patient's spinal cord region for a fifth stimulation period and according to a fifth stimulation schedule in which the fifth electrical signal is delivered for less than, e.g., 30 minutes per day. In this way, the method 400 can include incrementally reducing a patient's stimulation dose over subsequent stimulation periods. Any number of incremental stimulation periods can be used, including two, three, four, five, six, seven, eight, or more.

The first, second, and third electrical signals (and any other electrical signals delivered during subsequent stimulation periods) can have the same or generally similar parameters. For example, each of the first, second, and third electrical signals can have the same or generally the same frequency, pulse width, amplitude, and/or pulse dosing rate. Thus, in some embodiments the signal parameters remain the same during the first stimulation period, the second stimulation period, and the third stimulation period, and the only change is the duration that the signal is administered to the patient. In other embodiments, at least one parameter (e.g., frequency, pulse width, amplitude, and/or pulse dose) can change as the patient transitions from the first electrical signal to the second electrical signal, and/or from the second electrical signal to the third electrical signal, and so on.

In some embodiments, the first, second, and third electrical signals (and any other electrical signals delivered during subsequent stimulation periods) are paresthesia-free electrical signals that do not induce a sensation of paresthesia when administered to the patient. That is, the patient generally cannot sense whether the signal is being actively applied. As described in detail under Section 5.0, the paresthesia-free signals may include different types of paresthesia-free signals, including, but not limited to, high frequency, paresthesia-free signals and/or low frequency, paresthesia-free signals. In other embodiments, the one or more of the electrical signals delivered during operation of the method 400 can be paresthesia-producing signals.

Figure 5 is a block diagram illustrating another method 500 for treating a patient in accordance with embodiments of the present technology. Similar to the method 400 described with reference to Figure 4, some or all of the operations in the method 500 shown in Figure 5 can be performed by a processor executing instructions stored on one or more elements of a patient treatment system, including the patient treatment system 100 described with reference to Figure 1.

The method 500 can begin in block 502 by delivering a first electrical signal to the patient's spinal cord region via an implanted signal delivery device, for a first stimulation period according to a first stimulation schedule in which the first electrical signal is delivered for at least 12 hours per day, such as at least 16 hours per day or at least 20 hours per day. The operation at block 502 can the same as, or generally the same as, the operation at block 402 of the method 400 described with reference to Figure 4. The method 500 can continue in block 504 by delivering, after the first stimulation period, a second electrical signal to the patient's spinal cord region via the implanted signal delivery device, for a second stimulation period and according to a second stimulation schedule in which the second electrical signal is delivered for, e.g., less than 6 hours per day, such as less than 5 hours per day, less than 4 hours per day, etc. The operation at block 504 can be the same as, or generally the same as, the operation at block 402 of the method 400 described with reference to Figure 4.

The method 500 can continue in block 506 by determining whether a therapeutic effect obtained during the first stimulation period is maintained during the second stimulation period. For example, if patient pain is reduced relative to baseline during the first stimulation period, the operation at block 506 can include determining whether the reduction in patient pain is maintained during the second stimulation period. In such embodiments, determining whether patient pain is maintained during the second stimulation period can include comparing a VAS or NRS pain score during the first stimulation period to a VAS or NRS pain score during the second stimulation period. If the VAS or NRS pain score during the second stimulation period is no more than 20% greater than the VAS or NRS pain score during the first stimulation period, the reduction in pain is considered to be maintained. Of course, other thresholds (e.g., 0%, 10%, 30%, etc.) and/or other metrics (e.g., spontaneous neural activity, evoked potential, etc.) can be used to determine whether the therapeutic effect is maintained at block 506.

If the therapeutic effect is determined as being maintained at block 506, the method 500 can optionally continue at block 508 by reducing (1) the duration of individual stimulation sessions, and/or (2) the number of stimulation sessions per week. An example of reducing the duration of individual stimulation sessions includes transitioning from 45 minute stimulation sessions to 30 minute stimulation sessions. An example of reducing the number of stimulation sessions per weeks includes transitioning from one stimulation session per day to one stimulation session every other day. Of course, the foregoing are provided by way of example only-one skilled in the art will appreciate from the disclosure herein that the duration and/or number of stimulation sessions can be reduced within any of the ranges provided herein.

If the therapeutic effect is determined as not being maintained at block 506, the method 500 can continue at block 510 by increasing (1) the duration of individual stimulation sessions, and/or (2) the number of stimulation sessions per week. An example of increasing the duration of individual stimulation sessions includes transitioning from 45 minute stimulation sessions to 1 hour stimulation sessions. An example of increasing the number of stimulation sessions per week includes transitioning from one stimulation session per day to two stimulation sessions per day. As with the disclosed embodiments above, the foregoing are provided by way of example only-one skilled in the art will appreciate from the disclosure herein that the duration and/or number of stimulation sessions can be increased within any of the ranges provided herein.

In some embodiments, the operations in blocks 506, 508, and 510 can be iteratively performed until the lowest effective stimulation dose/schedule that maintains the therapeutic effect is discovered, such that the patient steps through a plurality of stimulation schedules. That is, the stimulation schedule/dose is titrated to (i) minimize power requirements, (ii) have the shortest stimulation sessions, (iii) have the fewest number of stimulation sessions per day, and/or (iv) have the longest duration between subsequent stimulation sessions, while maintaining the therapeutic effect of the stimulation therapy. For example, in some embodiments the stimulation schedule/dose can be iteratively titrated by repeating the operations in blocks 506 and 508 until a stimulation schedule is reached in which the patient receives a single, relatively short (e.g., less than about 1 hour) stimulation session per week. In some embodiments, it is expected that the patient may be titrated through two, three, four, five, six, seven, eight, nine, ten, or more stimulation schedules during the iterative process of repeating the operations in blocks 506, 508, and 510. Once the lowest effective stimulation dose/schedule is discovered, the operations in blocks 506, 508, and 510 can be periodically repeated to ensure that the stimulation schedule remains optimized.

In some embodiments, the methods 400 and 500 described with respect to Figures 4 and 5 are performed using a neurostimulation system that includes an implanted signal delivery device and an implanted signal generator having a primary cell battery. Use of a primary cell battery is more convenient than a secondary cell battery because it eliminates the need for the patient to recharge the device. However, use of a primary cell battery in implanted signal generators for high frequency stimulation has previously been unrealistic due to the relatively high power requirements of high frequency stimulation systems. In other words, the lifespan of the implanted device with a primary cell battery would not be long enough to justify the patient conveniences associated with a primary cell battery. However, stimulation delivered in accordance with the present technology, such as in accordance with the method 400 described with respect to Figure 4, is expected to reduce power consumption (e.g., by virtue of reducing the duration that stimulation is administered for) such that it becomes reasonable to utilize an implantable signal generator with a primary cell battery, eliminating the need for the patient to recharge the device.

In some embodiments, the methods 400 and 500 described with respect to Figures 4 and 5 are performed using a neurostimulation system that includes an implanted signal delivery device and an external signal generator. In such embodiments, the external signal generator can be a wearable device (e.g., a puck) that the patient attaches to their skin or otherwise places in alignment with the implanted signal delivery device during stimulation sessions. The patient can recharge the external signal generator during periods of nonuse. Of course, the methods 400 and 500 described with respect to Figures 4 and 5 can be performed by other stimulation systems, including any variation of the system 100 described with respect to Figure 1, and other stimulation systems known in the art.

### 5.0 Representative Stimulation Parameters

The electrical signals described herein, including the first, second, and third electrical signals described with respect to the method 400, can have a frequency in a frequency range of from about 0.01 Hz to about 1 MHz. For example, the electrical signal can be a "high frequency" electrical signal and have a frequency of from about 1.2 kHz to about 1 MHz, or from about 1.2 kHz to about 500 kHz, or from about 1.2 kHz to about 100 kHz, or from about 1.5 kHz to about 100 kHz, or from about 2 kHz to about 50 kHz, or from about 3 kHz to about 20 kHz, or from about 3 kHz to about 15 kHz, or from about 5 kHz to about 15 kHz, or from about 3 kHz to about 10 kHz, or 1.5 kHz, 2 kHz, 3 kHz, 4 kHz, 5 kHz, 10 kHz, 15 kHz, 20 kHz, 50 kHz, 100 kHz, or 500 kHz, or 1 MHz. Alternatively, the electrical signal can be a "low frequency" electrical signal and have a frequency of from about 0.01 Hz to about 1 kHz, or from about 1 Hz to about 500 Hz, or from about 1 Hz to about 100 Hz. The electrical signal may have a pulse width of from about 1 microsecond to about 2 seconds. For example, the electrical signal may have a pulse width of from about 1 microsecond to about 417 microseconds, or from about 10 microseconds to about 333 microseconds, or from about 10 microseconds to about 166 microseconds, or from about 25 microseconds to about 166 microseconds, or from about 20 microseconds to about 100 microseconds, or from about 30 microseconds to about 100 microseconds, or from about 30 microseconds to about 40 microseconds, or from about 10 microseconds to about 50 microseconds, or from about 20 microseconds to about 40 microseconds, or from about 25 microseconds to about 35 microseconds, or from about 30 microseconds to about 35 microseconds, or 30 microseconds. As additional examples, the electrical signal may have a pulse width of from about 5 milliseconds to about 2 seconds, or between about 5 milliseconds and about 1 second, or between about 100 milliseconds and about 1 second, or between about 100 milliseconds and about 500 milliseconds, or between about 200 milliseconds and about 500 milliseconds, or between about 250 milliseconds and about 400 milliseconds. In some embodiments, the electrical signal can be administered at current amplitudes of from 0.1 mA to 20 mA, or 0.5 mA to 10 mA, or 0.5 mA to 7 mA, or 0.5 mA to 5 mA. The electrical signal can also be administered according to a pulse dose rate ranging from about 10% to about 100%, such as about 10% to about 50%.

In many of the embodiments described herein, the electrical signals generally do not produce paresthesia when delivered to the patient, and can therefore be referred to as "non-paresthesia producing electrical signals," or "paresthesia-free signals." As set forth previously, such signals have combinations of frequency, pulse widths, amplitudes, and/or pulse dose rates that cause the signal to be below a patient's perception threshold. For example, some paresthesia-free electrical signals may have a frequency of between about 1 Hz and about 1 MHz, or between about 1.2 kHz and 500 kHz, or between about 1.2 kHz and about 100 kHz. As another example, some paresthesia-free electrical signals may be delivered in discrete bursts, separated by quiescent periods in which the electrical signal is not delivered. As yet another example, some paresthesia-free electrical signals may have a "long" pulse width of between about 5 milliseconds and about 2 seconds with an amplitude below an activation threshold of a target neural population. Additional examples of paresthesia-free electrical signals, including those mentioned above, are described in U.S. Patent Application Publication Nos. US2010/0274314, US2021/0228881, and US2022/0401730, and U.S. Provisional Patent Application No. 63/501 122.

In yet other embodiments, the electrical signals described herein may produce paresthesia when delivered to the patient, and can therefore be referred to as "paresthesia-producing" signals. Such signals have combinations of frequency, pulse widths, amplitudes, and/or pulse dosing rates that cause the signal to be above a patient's perception threshold.

### 6.0 Representative Clinical Applications

The present technology has generally been described in the context of treating pain. For example, stimulation delivered during the first stimulation period reduces patient pain, and stimulation delivered during the second stimulation period maintains the reduction in patient pain. Without being bound by theory, it is expected that many different types of pain can be treated using the stimulation schedules described herein. For example, the stimulation schedules described herein can be applied to treat pain at various locations, including neck pain, back pain, and/or leg pain. Similarly, the stimulation schedules described herein can be applied to treat pain associated with various neuropathies, such as diabetic neuropathy, peripheral neuropathy, peripheral polyneuropathy, chemotherapy-induced neuropathy, among others. Such pain is typically (but not always) located in a patient's arms, hands, legs, and/or feet. The stimulation schedules described herein can also be applied to treat other types of pain, including headache pain, facial pain, cancer pain, shoulder pain, elbow pain, hand pain, other upper extremity pain, abdominal pain, visceral pain, pelvic pain, hip pain, knee pain, foot pain, other lower extremity pain, whole body pain, phantom limb pain, among others. The stimulation schedules described herein can also be applied to treat different types of pain, including neuropathic pain and nociceptive pain.

The stimulation schedules described herein can also be used to treat indications or symptoms other than pain. For example, the stimulation schedules described herein can be used to treat sensory loss or numbness (e.g., sensation loss associated with diabetic neuropathy or other neuropathies, spinal cord injury, etc.). In such embodiments, stimulation delivered during the first stimulation period improves patient sensation/reduces patient numbness, and stimulation delivered during the second stimulation period maintains the improvement in patient sensation/reduction in patient numbness. The stimulation schedules described herein can also be used to treat abnormal sensations, such as naturally occurring tingling or paresthesia (e.g., paresthesia associated with diabetic neuropathy or other neuropathies).

In further embodiments, the stimulation schedules described herein can be used to treat neurodegenerative diseases, including Parkinson's diseases and related disorders, Alzheimer's diseases and related disorders, Prion disease, Motor neuron diseases, Huntington's disease, Spinocerebellar ataxia, Spinal muscular atrophy, Amyotrophic lateral sclerosis, Friedreich's ataxia, and Lewy body disease. The stimulation schedules can also be used to treat various other motor or movement disorders, such as tremor, epilepsy, or the like. In some embodiments, the stimulation schedules described herein can be used to treat blood glucose abnormalities, such as metabolic syndrome, diabetes (e.g., type 2 diabetes and/or type 1 diabetes), among others. In yet other embodiments, the stimulation schedules described herein can be used to treat cardiovascular disorders, such as heart failure (e.g., congestive heart failure, decompensated heart failure, etc.). Of course, the stimulation schedules described herein may be applied to treat indications and/or symptoms beyond those expressly recited herein.

### 7.0 Clinical Data

Nevro Corp., the applicant of the present application, is conducting clinical studies of certain reduced stimulation schedules described herein. One study included cohorts of both "already implanted patients" (e.g., patients who already had a stimulator implanted, and thus were already receiving continuous spinal cord stimulation for a prolonged duration, such as three months or more), and "naive patients" (e.g., patients who had the stimulator implanted to participate in the study, and thus were not previously receiving prolonged continuous stimulation). The already implanted patients and the naive patients were assigned to one of three groups: (1) group 1, which received 8 hours of daily stimulation, followed by 16 hours of no stimulation, (2) group 2, which received 4 hours of daily stimulation, followed by 20 hours of no stimulation, and (3) group 3, which received 2 hours of daily stimulation, followed by 22 hours of no stimulation. For all groups, the stimulation had a frequency of 10 kHz and was applied to the patient's spinal cord.

Figure 6 is a chart 600 showing the initial responder rate of already implanted patients and naive patients assigned to each group. A patient was considered a responder if the patient had greater than or equal to 50% reduction in pain relative to their baseline pain level within 7-14 days of receiving the reduced dose of stimulation. For both already implanted patients and naive patients, the baseline pain level was the patient's pain level (measured by VAS pain score) before receiving any stimulation therapy (or, for some already implanted patients, after an extended break from stimulation such that the patient's pain levels returned to pre-stimulation levels and an effective baseline state). Thus, for the already implanted patients, the baseline represented the patients' pain levels before the patient received continuous stimulation. As shown in FIG. 6, already implanted patients had a high responder rate in each group: 100% of already implanted patients receiving 8 hours of stimulation per day, 88% of already implanted patients receiving 4 hours of stimulation per day, and 93% of already implanted patients receiving 2 hours of stimulation per day experienced greater than or equal to a 50% reduction in pain relative to their baseline pain level. Naive patients also had relatively high responder rates, although generally lower than already implanted patients. For example, 83% of naive patients who received 8 hours of stimulation per day, 93% of naïve patients who received 4 hours of stimulation per day, and 75% of naïve patients who received 2 hours of stimulation per day experienced greater than or equal to a 50% reduction in pain relative to their baseline pain level.

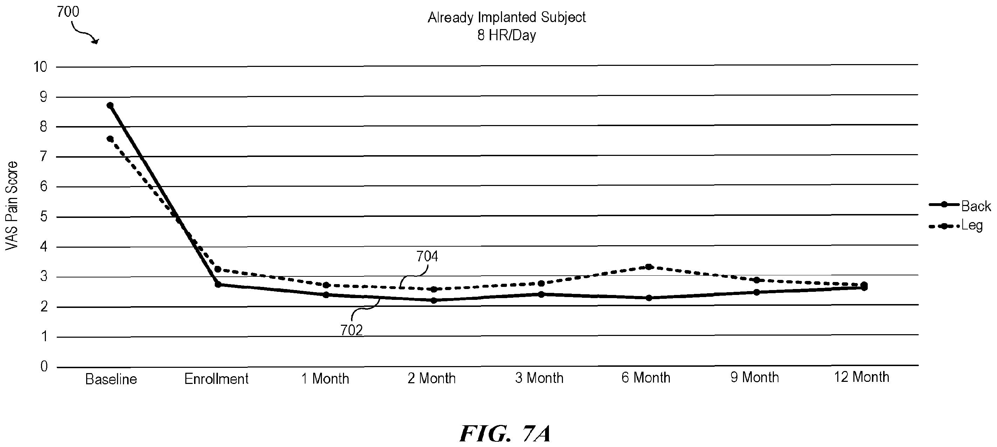

Figures 7A-7F are graphs showing the average reduction in pain levels for the patients included in the chart 600. Figure 7A is a graph 700 illustrating average back pain scores (line 702) and average leg pain scores (line 704) at various intervals for the cohort of already implanted patients assigned to group 1 (8 hours of stimulation per day). As shown on the x-axis, pain scores were measured at baseline, at enrollment, at 1 month post enrollment, at 2 months post enrollment, at 3 months post enrollment, at 6 months post enrollment, at 9 months post enrollment, and at 12 months post enrollment. As set forth above, baseline refers to patient pain level before the patient received stimulation therapy. Enrollment refers to when the patients began the trial-that is, when the patient transitioned from receiving continuous stimulation to 8 hours of stimulation per day. 3 months post enrollment represents the patient's pain level after being subjected to 8 hours of stimulation per day for 3 months, 6 months post enrollment represents the patient's pain level after being subjected to 8 hours of stimulation per day for 6 months, and so on. As shown in Figure 7A, continuous stimulation reduced both back and leg pain scores (e.g., comparing average pain scores reported at baseline to average pain scores reported at enrollment). Moreover, the 8 hours of daily stimulation substantially maintained back and leg pain scores at the 3 month, 6 month, 9 month, and 12 month endpoints.

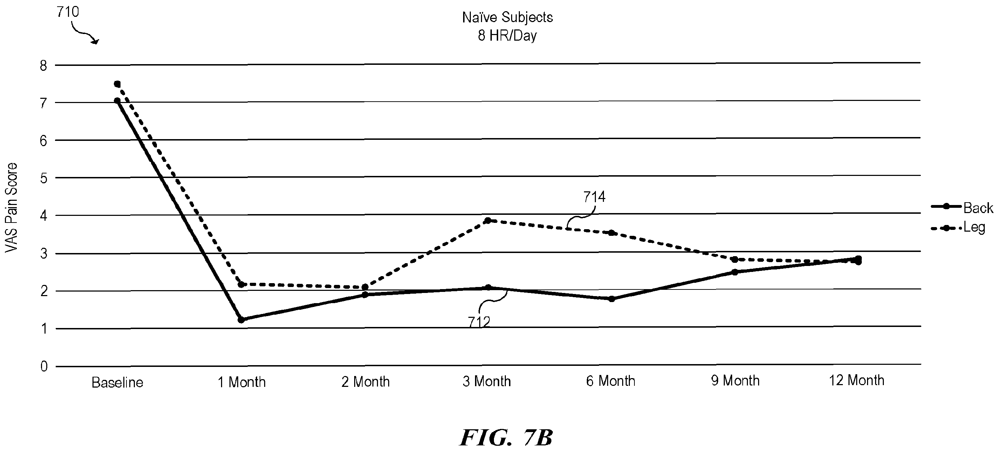

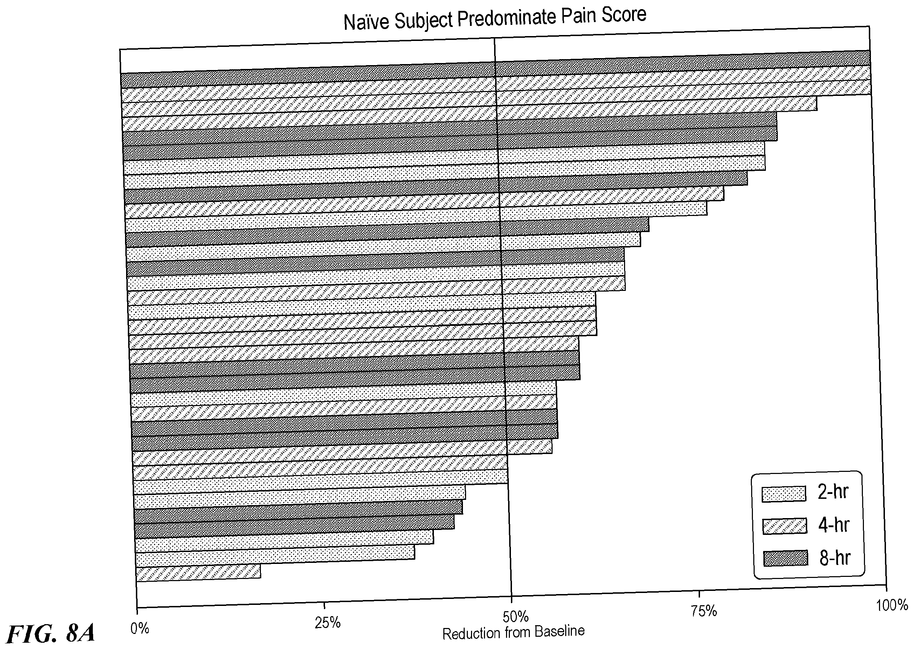

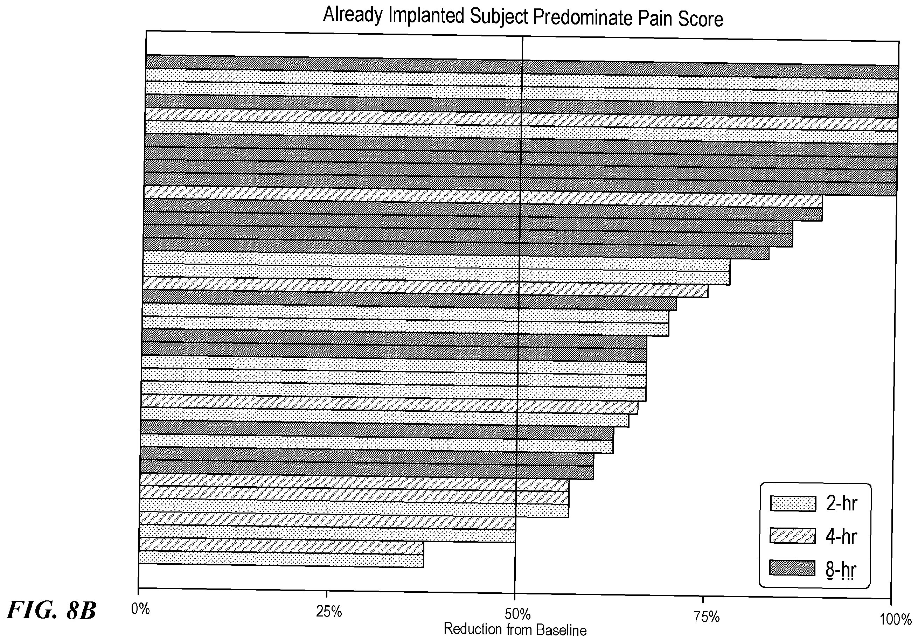

Figure 7B is a graph 710 comparing average back pain scores (line 712) and average leg pain scores (line 714) at various intervals for the cohort of naive patients assigned to group 1 (8 hours of stimulation per day). Unlike the graph 700, the graph 710 does not include an "enrollment" data set. Naive patients' pain scores at baseline are the same as their pain scores at enrollment because they did not previously receive continuous stimulation. As shown in Figure 7B, 8 hours of stimulation per day reduced both back and leg pain scores in naive patients. Without intending to be bound by theory, this means that, in at least some patients, it is not necessary to have an initial period of continuous stimulation that precedes the reduced dose, as described above in Section 4.0. However, some patients may respond better, may have a greater degree of pain relief, and/or may ultimately be able to be titrated to a lower level of therapy if they initially receive continuous stimulation, as also described above in Section 4.0.

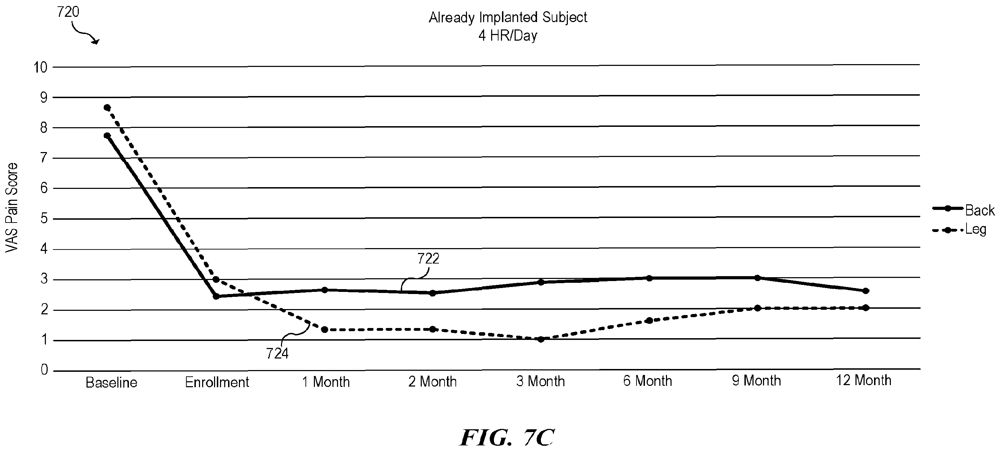

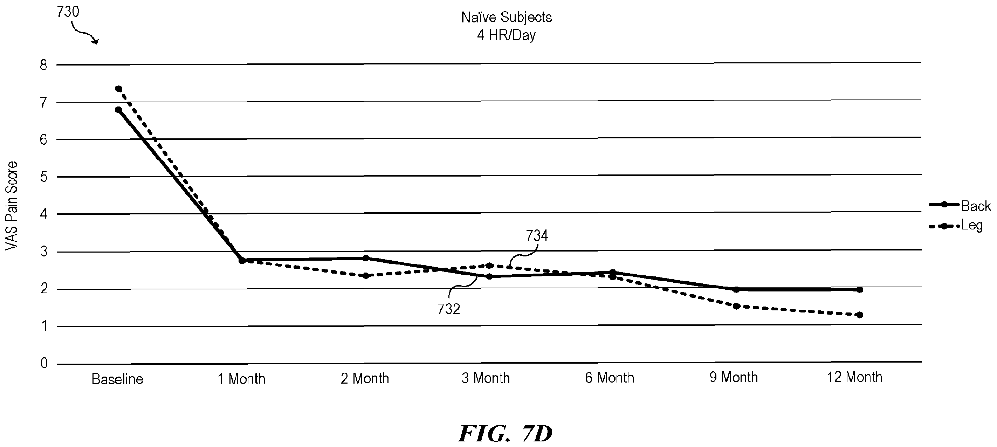

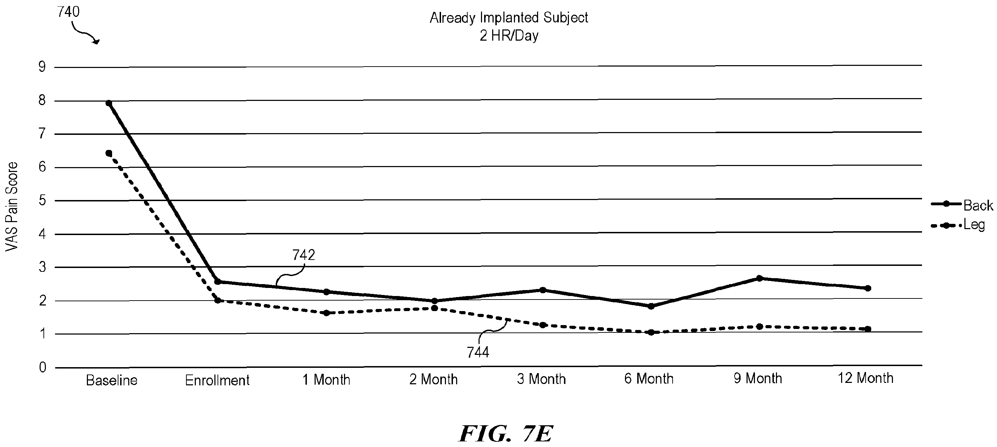

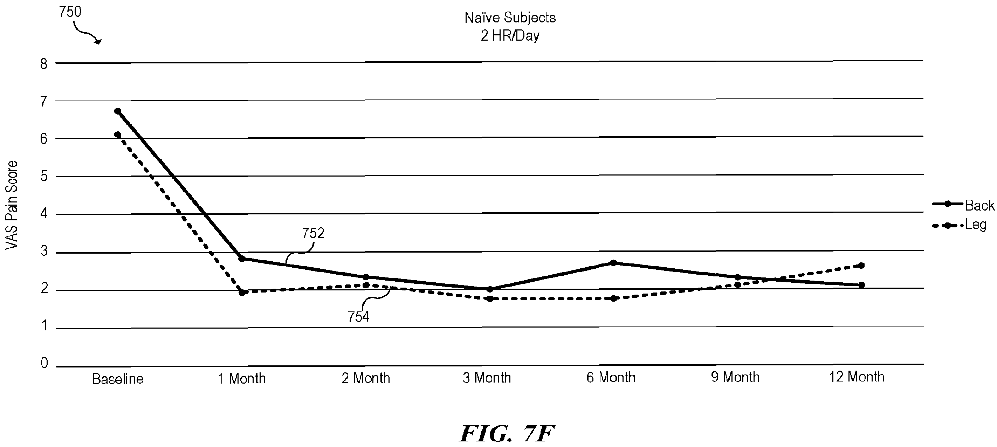

Figures 7C-7F show that back and leg pain was also substantially reduced relative to baseline pain levels in the patients assigned to group 2 (4 hours of stimulation per day) and group 3 (2 hours of stimulation per day). Figure 7C is a graph 720 comparing average back pain scores (line 722) and average leg pain scores (line 724) at baseline, at enrollment, and at various stages post-enrollment for the cohort of already implanted subjects assigned to group 2 (4 hours of stimulation per day). As shown in Figure 7C, 4 hours of stimulation per day reduced both back and leg pain scores in already implanted patients. Figure 7D is a graph 730 comparing average back pain scores (line 732) and average leg pain scores (line 734) at baseline/enrollment and at various stages post-enrollment for the cohort of naive subjects assigned to group 2 (4 hours of stimulation per day). As shown in Figure 7D, 4 hours of stimulation per day reduced both back and leg pain scores in naive patients. Figure 7E is a graph 740 comparing average back pain scores (line 742) and average leg pain scores (line 744) at baseline, at enrollment, and at various stages post-enrollment for the cohort of already implanted subjects assigned to group 3 (2 hours of stimulation per day). As shown in Figure 7E, 2 hours of stimulation per day reduced both back and leg pain scores in already implanted subjects. Finally, Figure 7F is a graph 750 comparing average back pain scores (line 750) and average leg pain scores (line 754) at baseline/enrollment and at various stages post-enrollment for the cohort of naïve subjects assigned to group 3 (2 hours of stimulation per day). As shown in Figure 7F, 2 hours of stimulation per day also reduced both back and leg pain scores in naïve patients.

Figures 8A and 8B illustrate individualized patient results from the same trials that produced the aggregated data reported above. In particular, Figure 8A is a tornado chart illustrating the magnitude of pain reduction relative to baseline (as a percent reduction from baseline) for each naive patient in groups 1-3, and Figure 8B is a tornado chart illustrating the magnitude of pain reduction relative to baseline (as a percent reduction from baseline) for each already implanted patient in groups 1-3. Referring collectively to Figures 8A and 8B, the x axis measures the percent reduction in pain from baseline, with 50% reduction the mid-point of the graph. Each horizontal bar represents an individual patient's reduction in pain, measured at 3-months post enrollment (for responders who continued with the trial) or at the failure point (for non-responders having less than 50% pain reduction). Because the trial was set to identify responders as patients with at least a 50% pain reduction relative to baseline, individual patients identified by horizontal bars that extend past the 50% mark were responders. Of note, in both the already implanted groups and naïve groups, at least some patients reported a 100% reduction in pain relative to baseline.

Taken together, the data in Figures 6-8B support that a reduced dose of stimulation can provide effective therapy. For example, Figures 6-8B demonstrate that 8 hours of daily stimulation, 4 hours of daily stimulation, and 2 hours of daily stimulation reduced, or at least maintained a reduction in, both back pain and leg pain. Although data is only reported for three particular stimulation schedules (i.e., 8 hours per day, 4 hours per day, and 2 hours per day), it is expected that other reduced stimulation schedules described herein will similarly provide effective therapy.

Moreover, the data in Figures 6-8B support that, for at least some patients, the reduced dose of stimulation can be effective regardless of whether it is preceded by a period of continuous stimulation. Without being bound by theory, it is expected that, in some patients, the reduced dose of therapy may be substantially as effective if the patient begins with the reduced dose, as opposed to starting with continuous therapy and transitioning to a reduced dose. In other patients, however, it is expected that the reduced dose of therapy may be more effective if it is preceded by a period of continuous stimulation. Accordingly, as described in detail in Section 4.0 above, the present technology includes (1) embodiments in which patients receive continuous or generally continuous stimulation for a first stimulation period before transitioning to a reduced dose of stimulation during a second stimulation period, and (2) embodiments in which the patients start therapy at a reduced dose of stimulation. In either embodiment, the dose can be further titrated down to reduce the number of stimulation sessions per day or per week, and/or to reduce the duration of stimulation sessions. Moreover, the foregoing data shows that a reduced dose of stimulation can maintain consistent pain relief over at least 12 months, which is expected to translate into substantially reduced power consumption and associated patient advantages.

### 9.0 Conclusion

From the foregoing, it will be appreciated that specific embodiments of the disclosed technology have been described herein for purposes of illustration, but that various modifications may be made without deviating from the technology. For example, therapy signals described herein can be delivered at combinations of parameter values within the foregoing ranges at values that are not expressly disclosed herein. Certain aspects of the technology described in the context of particular embodiments may be combined or eliminated in other embodiments. For example, the therapy signal can be monophasic with a passive charge elimination phase. In some embodiments, the foregoing techniques can be used to address patient deficits than pain. Further, while advantages associated with certain embodiments of the disclosed technology have been described in the context of those embodiments, other embodiments may also exhibit such advantages, and not all embodiments need necessarily exhibit such advantages to fall within the scope of the present technology. Accordingly, the disclosure and associated technology can encompass other embodiments not expressly shown or described herein.

The use of "and/or," as in "A and/or B" refers to A alone, B alone, and both A and B. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Where a range of values is provided, it is understood that each intervening value, to the tenth of the unit of the lower limit unless the context clearly dictates otherwise, to between the upper and lower limits of that range is also specifically disclosed. Each smaller range between any stated value or intervening value in a stated range and any other stated or intervening value in that stated range is encompassed within the disclosure. The upper and lower limits of these smaller ranges may independently be included or excluded in the range, and each range where either, neither or both limits are included in the smaller ranges is also encompassed within the disclosure, subject to any specifically excluded limit in the stated range. Where the stated range includes one or both of the limits, ranges excluding either or both of those included limits are also included in the disclosure.

## Claims

1. A patient treatment system, comprising:
an implantable signal delivery (15) device positionable proximate a target neural population in a patient; and
a signal generator (10) having a controller programmed with instructions that, when executed, cause the signal generator to:
deliver a first paresthesia-free electrical signal to the target neural population via the implanted signal delivery device for a first stimulation period and according to a first stimulation schedule in which the first paresthesia-free electrical signal is delivered for at least 12 hours per day, wherein the first paresthesia-free electrical signal at least partially alleviates one or more patient symptoms; and
after the first stimulation period, automatically deliver a second paresthesia-free electrical signal to the target neural population via the implanted signal delivery device for a second stimulation period and according to a second stimulation schedule in which the second paresthesia-free electrical signal is delivered for less than 6 hours per day, wherein the second paresthesia-free electrical signal at least partially maintains the alleviation of the one or more patient symptoms achieved during the first stimulation period.

2. The system of claim 1, wherein the first stimulation period has a predetermined duration, and wherein the instructions, when executed, cause the signal generator to automatically cease delivery of the first electrical signal and initiate delivery of the second electrical signal after the predetermined duration.

3. The system of claim 2, wherein the predetermined duration is between about 1 month and about 1 year.

4. The system of claim 2, wherein the predetermined duration is between about 1 month and about 6 months.

5. The system of claim 1, wherein the instructions, when executed, cause the signal generator to automatically cease delivery of the first electrical signal and initiate delivery of the second electrical signal in response to a user input.

6. The system of claim 1, wherein the instructions, when executed, cause the signal generator to automatically cease delivery of the first electrical signal and initiate delivery of the second electrical signal in response to one or more criteria meeting one or more predetermined thresholds.

7. The system of claim 6, wherein the one or more criteria include evoked potential, spontaneous potential, patient activity level, and/or VAS pain score.

8. The system of claim 1, wherein the signal generator is an implantable signal generator.

9. The system of claim 8, wherein the signal generator comprises a primary cell battery.

10. The system of claim 1, wherein the signal generator is an external signal generator.

11. The system of claim 1, wherein the controller is internal to the signal generator.

12. The system of claim 1, wherein the controller is external to the signal generator.

13. The system of claim 1, wherein the instructions, when executed, further cause the signal generator to:
after the second stimulation period, automatically deliver a third paresthesia-free electrical signal to the patient's spinal cord region via the implanted signal delivery device for a third stimulation period and according to a third stimulation schedule in which the third electrical signal is delivered for less than 2 hours per day, wherein the third electrical signal at least partially maintains the alleviation of the one or more patient symptoms achieved during the first and second stimulation periods.

## Patentansprüche

1. Patientenbehandlungssystem, umfassend:
- eine implantierbare Signalabgabevorrichtung (15), die in der Nähe einer Zielneuronenpopulation in einem Patienten positionierbar ist; und
- einen Signalgenerator (10) mit einer Steuerung, die mit Anweisungen programmiert ist, die bei Ausführung bewirken, dass der Signalgenerator:
- ein erstes parästhesiefreies elektrisches Signal an die Zielneuronenpopulation über die implantierte Signalabgabevorrichtung für einen ersten Stimulationszeitraum und nach einem ersten Stimulationsplan abgibt, bei dem das erste parästhesiefreie elektrische Signal zumindest 12 Stunden pro Tag abgegeben wird, wobei das erste parästhesiefreie elektrische Signal ein oder mehrere Symptome des Patienten zumindest teilweise lindert; und
- nach dem ersten Stimulationszeitraum automatisch ein zweites parästhesiefreies elektrisches Signal an die Zielneuronenpopulation über die implantierte Signalabgabevorrichtung für einen zweiten Stimulationszeitraum und nach einem zweiten Stimulationsplan abgibt, bei dem das zweite parästhesiefreie elektrische Signal weniger als 6 Stunden pro Tag abgegeben wird, wobei das zweite parästhesiefreie elektrische Signal die während des ersten Stimulationszeitraums erzielte Linderung eines oder mehrerer Patientensymptome zumindest teilweise aufrechterhält.

2. System nach Anspruch 1, wobei der erste Stimulationszeitraum eine vorbestimmte Dauer aufweist und wobei die Anweisungen bei Ausführung bewirken, dass der Signalgenerator die Abgabe des ersten elektrischen Signals automatisch einstellt und die Abgabe des zweiten elektrischen Signals nach der vorbestimmten Dauer einleitet.

3. System nach Anspruch 2, wobei die vorbestimmte Dauer zwischen etwa 1 Monat und etwa 1 Jahr liegt.

4. System nach Anspruch 2, wobei die vorbestimmte Dauer zwischen etwa 1 Monat und etwa 6 Monaten liegt.

5. System nach Anspruch 1, wobei die Anweisungen bei Ausführung bewirken, dass der Signalgenerator die Abgabe des ersten elektrischen Signals automatisch einstellt und die Abgabe des zweiten elektrischen Signals als Reaktion auf eine Benutzereingabe einleitet.

6. System nach Anspruch 1, wobei die Anweisungen bei Ausführung bewirken, dass der Signalgenerator die Abgabe des ersten elektrischen Signals automatisch einstellt und die Abgabe des zweiten elektrischen Signals als Reaktion auf ein oder mehrere Kriterien einleitet, die einen oder mehrere vorbestimmte Schwellenwerte erfüllen.

7. System nach Anspruch 6, wobei ein oder mehrere Kriterien evoziertes Potenzial, spontanes Potenzial, ein Aktivitätsniveau des Patienten und/oder einen VAS-Schmerzscore umfassen.

8. System nach Anspruch 1, wobei der Signalgenerator ein implantierbarer Signalgenerator ist.

9. System nach Anspruch 8, wobei der Signalgenerator eine Primärzellenbatterie aufweist.

10. System nach Anspruch 1, wobei der Signalgenerator ein externer Signalgenerator ist.

11. System nach Anspruch 1, wobei die Steuerung innerhalb des Signalgenerators ist.

12. System nach Anspruch 1, wobei die Steuerung außerhalb des Signalgenerators ist.

13. System nach Anspruch 1, wobei die Anweisungen bei Ausführung ferner bewirken, dass der Signalgenerator:
- nach dem zweiten Stimulationszeitraum automatisch ein drittes, parästhesiefreies elektrisches Signal an den Rückenmarksbereich des Patienten über die implantierte Signalabgabevorrichtung für einen dritten Stimulationszeitraum und nach einem dritten Stimulationsplan abgibt, bei dem das dritte elektrische Signal weniger als 2 Stunden pro Tag abgegeben wird, wobei das dritte elektrische Signal die während des ersten und zweiten Stimulationszeitraums erzielte Linderung eines oder mehrerer Patientensymptome zumindest teilweise aufrechterhält.

## Revendications

1. Système de traitement d'un patient, comprenant :
un dispositif implantable (15) de délivrance de signaux pouvant être positionné à proximité d'une population neuronale cible chez un patient ; et
un générateur de signaux (10) comportant un dispositif de commande programmé avec des instructions qui, lorsqu'elles sont exécutées, amènent le générateur de signaux à :
délivrer un premier signal électrique sans paresthésie à la population neuronale cible par l'intermédiaire du dispositif de délivrance de signaux implanté, pendant une première période de stimulation et selon un premier programme de stimulation dans lequel le premier signal électrique sans paresthésie est délivré pendant au moins 12 heures par jour, dans lequel le premier signal électrique sans paresthésie soulage au moins partiellement un ou plusieurs symptômes du patient ; et
après la première période de stimulation, délivrer automatiquement un deuxième signal électrique sans paresthésie à la population neuronale cible par l'intermédiaire du dispositif de délivrance de signaux implanté, pendant une deuxième période de stimulation et selon un deuxième programme de stimulation dans lequel le deuxième signal électrique sans paresthésie est délivré pendant moins de 6 heures par jour, dans lequel le deuxième signal électrique sans paresthésie maintient au moins partiellement le soulagement desdits un ou plusieurs symptômes du patient obtenu au cours de la première période de stimulation.

2. Système selon la revendication 1, dans lequel la première période de stimulation a une durée prédéterminée, et dans lequel les instructions, lorsqu'elles sont exécutées, amènent le générateur de signaux à cesser automatiquement la délivrance du premier signal électrique et à déclencher la délivrance du deuxième signal électrique à l'issue de la durée prédéterminée.

3. Système selon la revendication 2, dans lequel la durée prédéterminée est comprise entre environ 1 mois et environ 1 an.

4. Système selon la revendication 2, dans lequel la durée prédéterminée est comprise entre environ 1 mois et environ 6 mois.

5. Système selon la revendication 1, dans lequel les instructions, lorsqu'elles sont exécutées, amènent le générateur de signaux à cesser automatiquement la délivrance du premier signal électrique et à déclencher la délivrance du deuxième signal électrique en réponse à une commande de l'utilisateur.

6. Système selon la revendication 1, dans lequel les instructions, lorsqu'elles sont exécutées, amènent le générateur de signaux à cesser automatiquement la délivrance du premier signal électrique et à déclencher la délivrance du deuxième signal électrique en réponse au fait qu'un ou plusieurs critères atteignent un ou plusieurs seuils prédéterminés.

7. Système selon la revendication 6, dans lequel le ou les critères comprennent un potentiel évoqué, un potentiel spontané, le niveau d'activité du patient et/ou le score de douleur sur l'échelle EVA.

8. Système selon la revendication 1, dans lequel le générateur de signaux est un générateur de signaux implantable.

9. Système selon la revendication 8, dans lequel le générateur de signaux comprend une pile primaire.

10. Système selon la revendication 1, dans lequel le générateur de signaux est un générateur de signaux externe.

11. Système selon la revendication 1, dans lequel le dispositif de commande est interne au générateur de signaux.

12. Système selon la revendication 1, dans lequel le dispositif de commande est externe au générateur de signaux.

13. Système selon la revendication 1, dans lequel les instructions, lorsqu'elles sont exécutées, amènent en outre le générateur de signaux à :
après la deuxième période de stimulation, délivrer automatiquement un troisième signal électrique sans paresthésie à la région de la moelle épinière du patient par l'intermédiaire du dispositif de délivrance de signaux implanté, pendant une troisième période de stimulation et selon un troisième programme de stimulation dans lequel le troisième signal électrique est délivré pendant moins de 2 heures par jour, dans lequel le troisième signal électrique maintient au moins partiellement le soulagement desdits un ou plusieurs symptômes du patient obtenu au cours des première et deuxième périodes de stimulation.
